# EUROPEAN PATENT SPECIFICATION

(11) **EP 2 252 373 B1**
(45) Date of publication and mention of the grant of the patent: **13.06.2012**
(21) Application number: 09708378.6
(22) Date of filing: 05.02.2009
(51) Int. Cl.: A61P 35/00, C12N 15/11

(54) **NOVEL FER -LIKE PROTEIN, PHARMACEUTICAL COMPOSITIONS CONTAINING IT AND METHOD FOR ITS USE**
NEUES FER-ARTIGES PROTEIN, DIESES ENTHALTENDE PHARMAZEUTISCHE ZUSAMMENSETZUNGEN UND ANWENDUNGSVERFAHREN
NOUVELLE PROTÉINE DE TYPE FER, COMPOSITIONS PHARMACEUTIQUES LA CONTENANT ET PROCÉDÉ POUR SON UTILISATION

(30) Priority: 05.02.2008 US 6897 P; 05.06.2008 US 129112 P
(43) Date of publication of application: 24.11.2010
(73) Proprietor: Urifer Ltd, Raanana 43613 (IL)
(72) Inventor: NIR, Uri, 73130 Moshav Gamzo (IL); MAKOVSKY, Adar, 56000 Yahood (IL); SHPUNGIN, Sally, 52275 Ramat-Gan (IL)
(74) Representative: O'Connell, Maura
(86) International application number: PCT/IL2009/000136
(87) International publication number: WO 2009/098690

(56) References cited:
- WO-A-2007/104011
- WO-A-2007/107991
- WO-A-2008/066498
- HAO Q-L ET AL: "ISOLATION AND SEQUENCE ANALYSIS OF A NOVEL HUMAN TYROSINE KINASE GENE" MOLECULAR AND CELLULAR BIOLOGY, AMERICAN SOCIETY FOR MICROBIOLOGY, WASHINGTON, US, vol. 9, no. 4, 1 April 1989 (1989-04-01), pages 1587-1593, XP001070722 ISSN: 0270-7306 & WO 2008/066498 A (AGENCY SCIENCE TECH & RES [SG]; ULLRICH AXEL [DE]; RUHE JENS [SG]; HAR) 5 June 2008 (2008-06-05)
- SUZUKI YUTAKA ET AL: "Sequence comparison of human and mouse genes reveals a homologous block structure in the promoter regions." GENOME RESEARCH SEP 2004, vol. 14, no. 9, September 2004 (2004-09), pages 1711-1718, XP002548480 ISSN: 1088-9051
- SCHEETZ TODD E ET AL: "Large-scale gene discovery in human airway epithelia reveals novel transcripts." PHYSIOLOGICAL GENOMICS 12 MAR 2004, vol. 17, no. 1, 12 March 2004 (2004-03-12), pages 69-77, XP002548481 ISSN: 1531-2267
- PORCEL BETINA M ET AL: "Numerous novel annotations of the human genome sequence supported by a 5'-end-enriched cDNA collection." GENOME RESEARCH MAR 2004, vol. 14, no. 3, March 2004 (2004-03), pages 463-471, XP002548482 ISSN: 1088-9051

## Description

### FIELD OF THE INVENTION

This invention relates to pharmaceutical compositions, and more specifically to such compositions for the treatment of cancer.

### BACKGROUND OF THE INVENTION

The following prior art publications are considered as being relevant for an understanding of the invention.
Allard P, Zoubeidi A, Nguyen LT, Tessier S, Tanguay S, Chevrette M, Aprikian A and Chevalier S. (2000). Mol. Cell. Endocrinol., 159, 63-77.
Ben-Dor I, Bern O, Tennenbaum T and Nir U. (1999). Cell Growth Differ., 10, 113-129.
Craig AW, Zirngibl R, Williams K, Cole LA and Greer PA. (2001). Mol. Cell. Biol., 21, 603-613.
Delfino FJ, Stevenson H and Smithgall TE. (2006). J. Biol. Chem., 281, 8829-8835. Fischman K, Edman JC, Shackleford GM, Turner JA, Rutter WJ and Nir U. (1990). Mol. Cell. Biol., 10, 146-153.
Gascoigne KE and Taylor SS. (2008). Cancer Cell, 14, 111-122.
Greer P. (2002). Nat. Rev. Mol. Cell Biol., 3, 278-289.
Halachmy S, Bern O, Schreiber L, Carmel M, Sharabi Y, Shoham J and Nir U. (1997). Oncogene, 14, 2871-2880.
Hao Q-L, Heisterkamp N and Groffen J. (1989). Mol. Cell. Biol., 9, 1587-1593.
Hazan B, Bern O, Carmel M, Lejbkowicz F, Goldstein RS and Nir U. (1993). Cell Growth Differ., 4, 443-449.
Kaufmann SH, Desnoyers S, Ottaviano Y, Davidson NE and Poirier GG. (1993). Cancer Research, □□, 3976-3985.
Keshet E, Itin A, Fischman K and Nir U. (1990). Mol. Cell. Biol., 10, 5021-5025.
Letwin K, Yee S-P and Pawson T. (1988). Oncogene, 3, 621-627.
Orlovsky K, Ben-Dor I, Priel-Halachmi S, Malovany H and Nir U. (2000). Biochemistry, 39, 11084-11091.
Pasder O, Shpungin S, Salem Y, Makovsky A, Vilchick S, Michaeli S, Malovani H and Nir U. (2006). Oncogene, 25, 4194-4206.
Polyak K, Waldman T, He TC, Kinzler KW and Vogelstein B. (1996). Genes Dev., 10, 1945-1952.
Priel-Halachmi S, Ben-Dor I, Shpungin S, Tennenbaum T, Molavani H, Bachrach M, Salzberg S and Nir U. (2000). J. Biol. Chem., 275, 28902-28910.
Salem Y, Shpungin S, Pasder O, Pomp O, Taler M, Malovani H and Nir U. (2005). Cell Signal., 17, 341-353.
Sangrar W, Gao Y, Scott M, Truesdell P and Greer PA. (2007). Mol. Cell Biol., 27, 6140-6152.
Sangrar W, Zirgnibl RA, Gao Y, Muller WJ, Jia Z and Greer PA. (2005). Cancer Res., 65,3518-3522.
van Engeland M, Ramaekers FC, Schutte B and Reutelingsperger CP. (1996). Cytometry, □□, 131-139.

Fer is an intracellular tyrosine kinase which was found to reside in both the cytoplasm and nucleus of mammalian cells (Ben-Dor et al., 1999; Hao et al., 1989; Letwin et al., 1988). Together with c-Fes, Fer represents a distinct subfamily of intracellular tyrosine kinases that share a unique structure. Both kinases bear an extended N-terminal tail which contains an Fps/Fes/Fer/CIP4 homology domain (FCH), followed by three coiled-coil-forming regions (Greer, 2002). A truncated variant of Fer, termed FerT, uniquely accumulates in meiotic spermatogenic cells (Fischman et al., 1990; Hazan et al., 1993; Keshet et al., 1990). Fer and FerT share common kinase and SH2 domains but they differ in their N-terminal tail where the 412 amino-acid-long tail in Fer is replaced with a unique 43 amino-acid-long tail in FerT (Priel-Halachmi et al., 2000).

Although present in a wide variety of tissues and cells, mice devoid of active Fer develop normally, and the proliferation of fibroblasts derived from these mice is not impaired *in vitro* (Craig et al., 2001). However, Fer has been implicated in the response of cells to stress cues. Fer was shown to rescue cells from ionic radiation (Halachmy et al., 1997), to support their growth under oxygen deprivation (Salem et al., 2005), and to mediate the migratory response of fibroblasts to reactive oxygen species (Sangrar et al., 2007). The role of Fer in supporting cell growth under abnormal conditions is further manifested in malignant cells. Several lines of evidence suggest a supportive role of Fer in the progression and growth of malignant tumors. Fer was detected in all human malignant cell lines analyzed (Hao et al., 1989; Orlovsky et al., 2000) and its levels in malignant prostate tumors are significantly higher than those detected in benign tumors (Allard et al., 2000). Furthermore, down-regulation of Fer impairs the proliferation of prostate and breast carcinoma cells (Pasder et al., 2006) and abolishes the ability of prostate carcinoma PC3 cells to form colonies in soft agar (Allard et al., 2000). This pro-oncogenic activity of Fer differs from the tumor suppressive activity of C-Fes in colon cancer (CC) cells (Delfino et al., 2006; Sangrar et al., 2005).

### SUMMARY OF THE INVENTION

The present invention is based on the novel and unexpected finding of a novel Fer-like protein, referred to herein as "*FerC*" (Fer colorectal cancer). FerC is a 47kDa protein having the sequence SEQ ID NO: 12 that contains the SH2 and kinase domains of Fer, but has a novel N-terminal sequence (SEQ ID NO: 14). FerC was found to be present in six colon cancer cell-lines analyzed, and in the hepatocarcinoma (liver cancer) cell-lines: SK-Hep-1, Hep-G2, Huh-6, Huh-7 and Hep-3B. FerC was not detected in HT29 adenocarcinoma cells, CCD33 normal colon epithelial cells or normal human and mouse fibroblasts.

Depletion of FerC impairs cell-cycle progression and induces apoptotic death in treated colon cancer (CC) cells, an effect that was exacerbated by the simultaneous knockdown of both FerC and Fer. Cell viability in HCT116 cell cultures following depletion of one or both of the Fer and FerC proteins indicated that simultaneous knockdown of Fer and FerC decreases by two fold the percentage of viable cells in the treated cultures.

Knockdown of FerC interfered with cell-cycle progression in HCT116 and RKO cells, but also induced apoptotic cell death, manifested by increased accumulation of the sub-G1 population in the treated cells, an effect that was enhanced upon the simultaneous knockdown of Fer and FerC.

In one of its aspects, the invention provides a nucleotide sequence comprising a sequence that is antisense to at least a portion of the nucleotide sequence SEQ ID NO: 13 and capable of specifically binding to SEQ ID NO. 13 characterized in that the antisense nucleotide sequence is SEQ ID NO: 9. The invention further provides a pharmaceutical composition comprising a nucleotide sequence of the invention that is antisense to at least a portion of the nucleotide sequence SEQ ID NO: 13 and capable of specifically binding to SEQ ID NO. 13, and a physiologically acceptable carner. The pharmaceutical composition may be used in the treatment of various cancers, in particular, colorectal cancer and liver cancer. The pharmaceutical composition of the invention may further comprise a nucleotide sequence comprising a sequence that is antisense to at least a portion of the fer gene. Preferably, the sequence that is antisense to at least a portion of the fer gene is SEQ ID NO 8.

In yet another of its aspect, the invention provides an *in vitro* method for identifying a cancerous state in a cell comprising detecting FerC in the cell. The method preferably comprises:
(a) extracting proteins from the cell;
(b) detecting Fer94 in the protein extract using a first antibody reactive with Fer94 and FerC;
(c) detecting Fer94 in the protein extract using a second antibody reactive with Fer94 and not reactive with FerC;
the presence of a protein in the extract reactive to the first antibody and not reactive to the second antibody being indicative of the presence of FerC in the cell.

The second antibody preferably binds to an epitope located at the N-terminus of Fer94. The invention also provides a kit for identifying a cancerous state in a cell comprising:
(a) a first antibody reactive with Fer94 and FerC;
(b) a second antibody reactive with Fer94 and not reactive with FerC. antisense to at least a portion of the fer gene. Preferably, the sequence that is antisense to at least a portion of the fer gene is SEQ ID NO 8.

In yet another of its aspect, the invention provides a method for identifying a cancerous state in a cell comprising detecting FerC in the cell. The method preferably comprises:
(a) extracting proteins from the cell;
(b) detecting Fer94 in the protein extract using a first antibody reactive with Fer94 and FerC;
(c) detecting Fer94 in the protein extract using a second antibody reactive with Fer94 and not reactive with FerC;
the presence of a protein in the extract reactive to the first antibody and not reactive to the second antibody being indicative of the presence of FerC in the cell.

The second antibody preferably binds to an epitope located at the N-terminus of Fer94. The invention also provides a kit for identifying a cancerous state in a cell comprising:
(a) a first antibody reactive with Fer94 and FerC;
(b) a second antibody reactive with Fer94 and not reactive with FerC.

The invention further provides a method of treating cancer comprising administering to an individual a pharmaceutical composition of the invention. The cancer may be, for example, colorectal cancer, or liver cancer.

### BRIEF DESCRIPTION OF THE DRAWINGS

In order to understand the invention and to see how it may be carried out in practice, embodiments will now be described, by way of non-limiting example only, with reference to the accompanying drawings, in which:

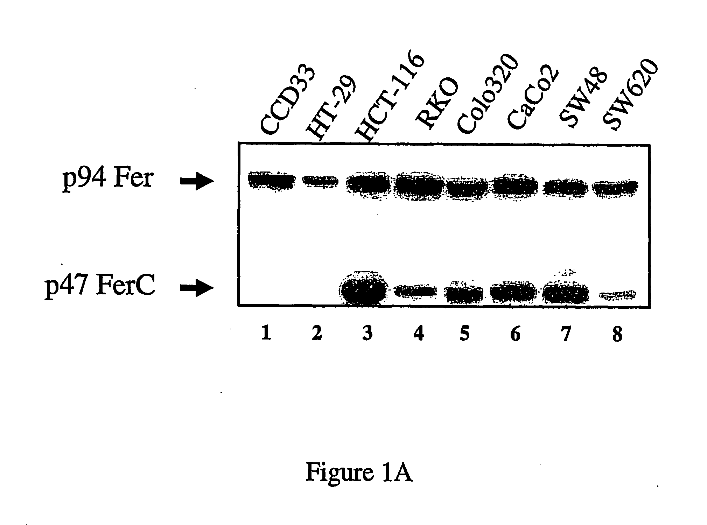

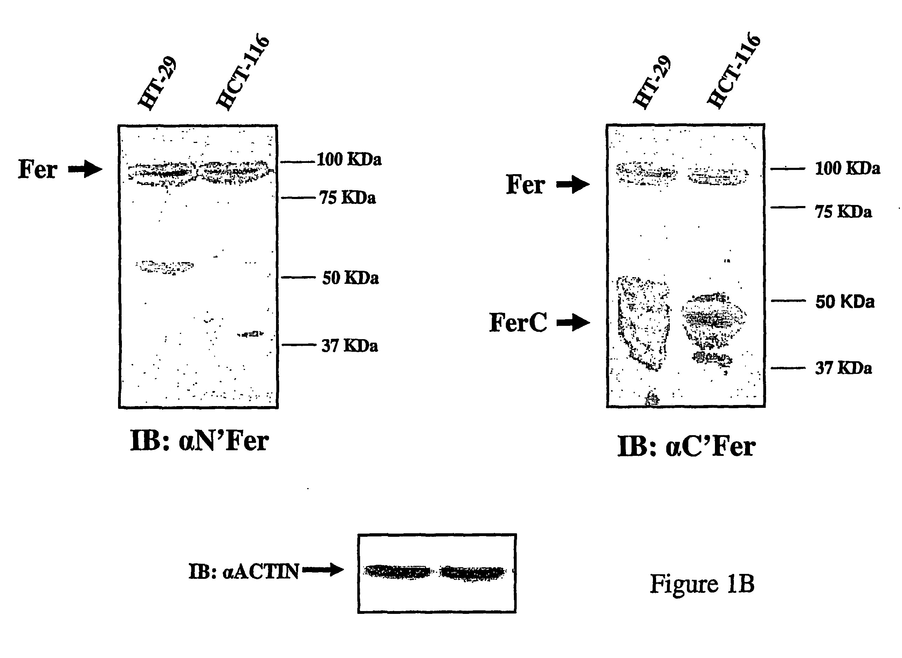

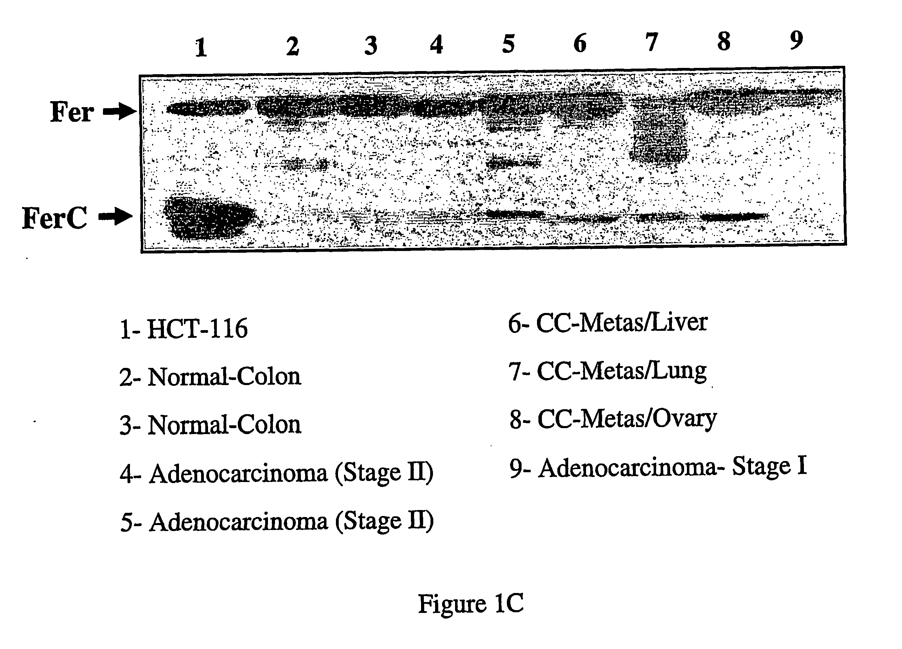

**Fig. 1** **A** shows western blot analysis of whole cell lysates of normal human colon epithelial cells-CCD33 (lane 1), and from seven other human CC-lines (lanes 2-8) after reaction of SDS-PAGE resolved proteins with anti-SH2-Fer antibodies; **Fig. 1** **B** shows western blot analysis of lysates from HT29 and from HCT116 of SDS-PAGE resolved proteins reacted with antibodies directed toward the unique N-terminal tail of Fer (1-189 aa) (left panel), or toward the C-terminal end of Fer (right panel); **Fig. 1C** shows.
detection with anti-SH2-Fer antibodies of: HCT116 whole cell lysates (1); normal human colon tissue (2,3); stage II primary colorectal adenocarcinoma (4,5); CC metastases to the liver (6); CC metastases to the lung (7); CC metastases to the ovary (8); and stage I adenocarcinoma (9);

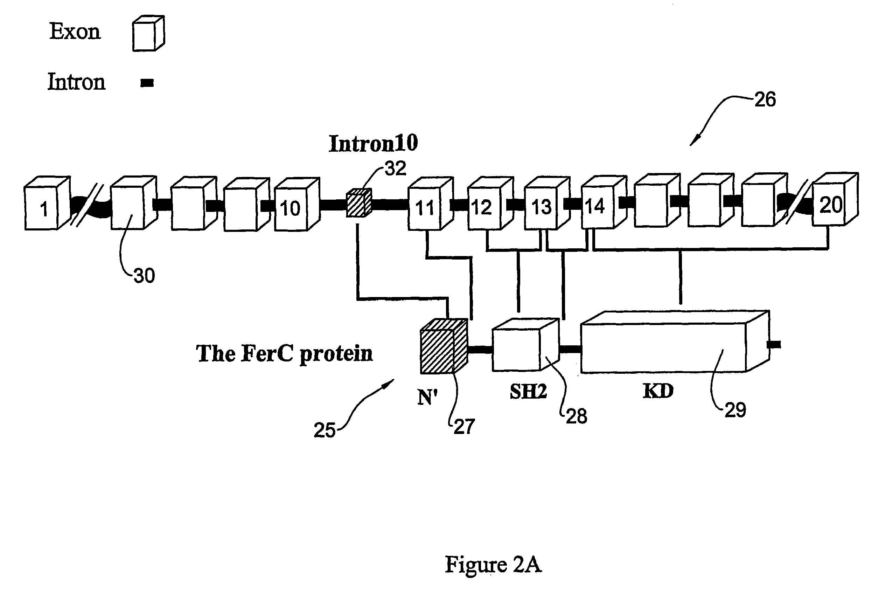

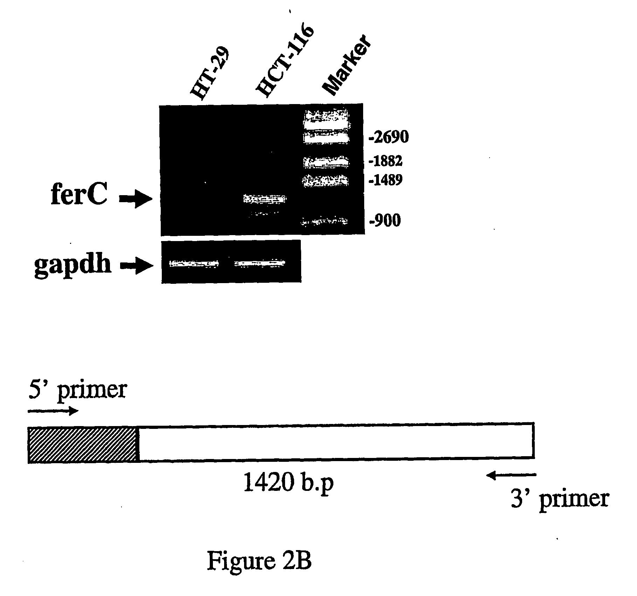

**Fig. 2** **A** shows schematically the structure of the FerC protein, and Fig. **2** **B** shows an RT-PCR product of 1420 bp that demonstrates the preferential accumulation of ferC mRNA in HCT116 cells;

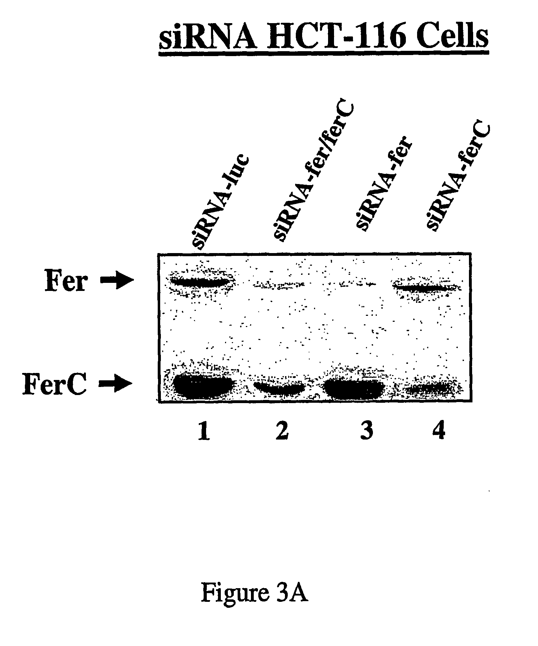

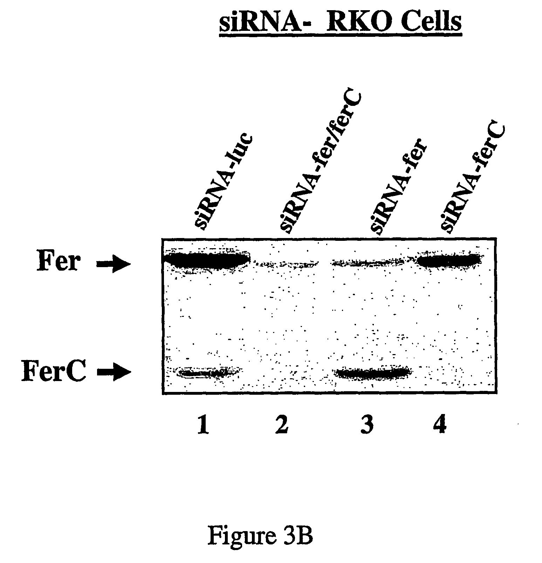

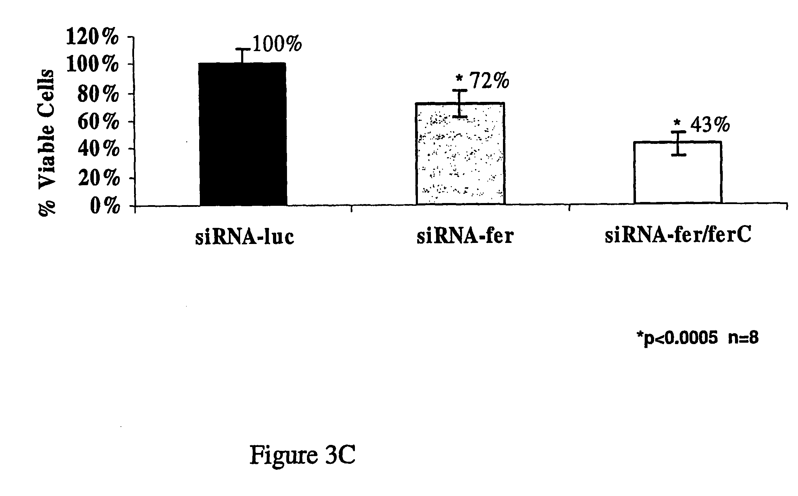

**Fig. 3** **A** shows knock down of Fer and **Fig. 3** **B** shows FerC, in HCT116 and RKO CC cells using 50nM specific and selective siRNAs; and **Fig. 3** **C** shows transfection of HCT116 with different siRNAs and evaluation by the XTT cell viability kit (Biol. Ind., Israel);

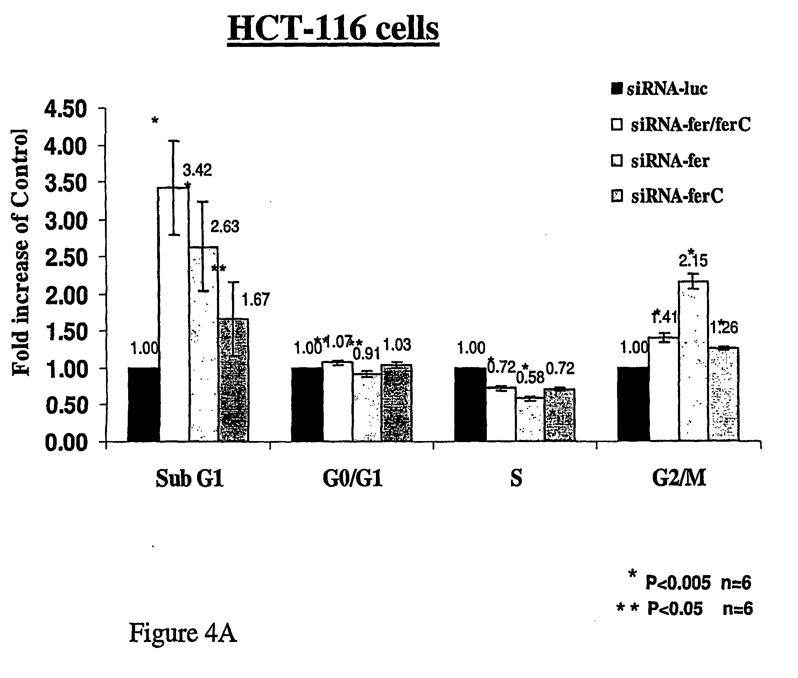

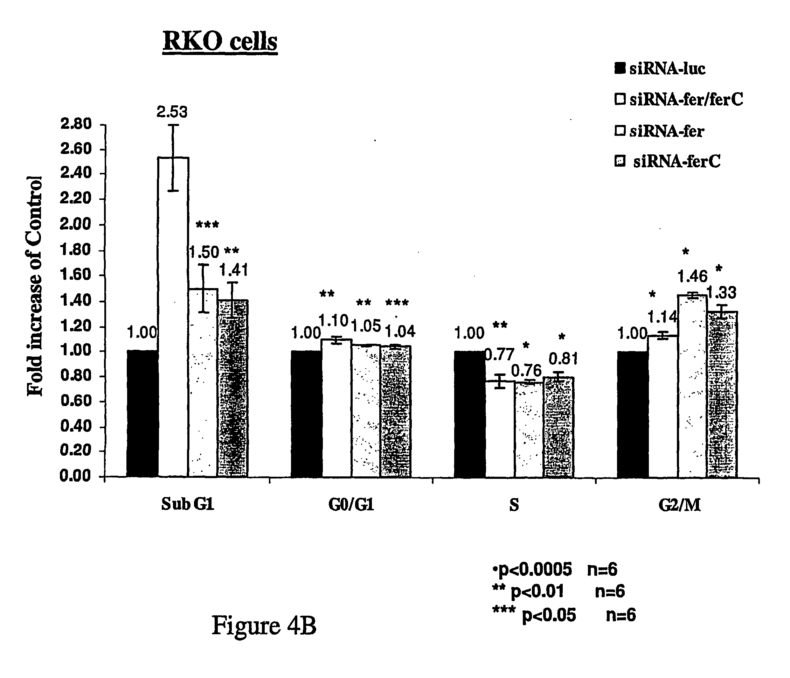

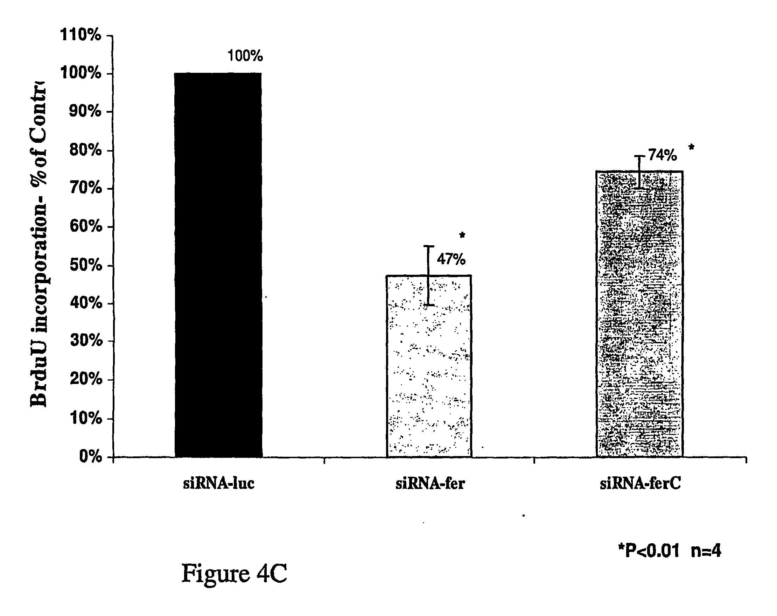

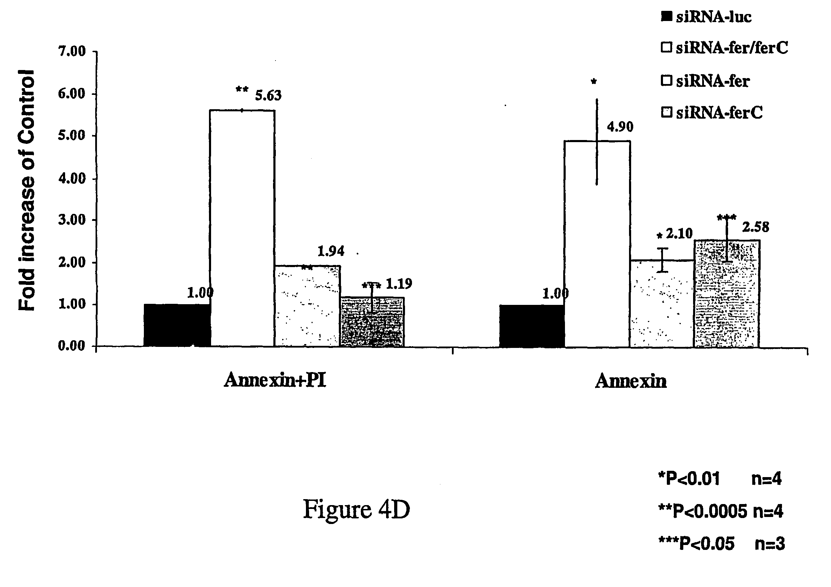

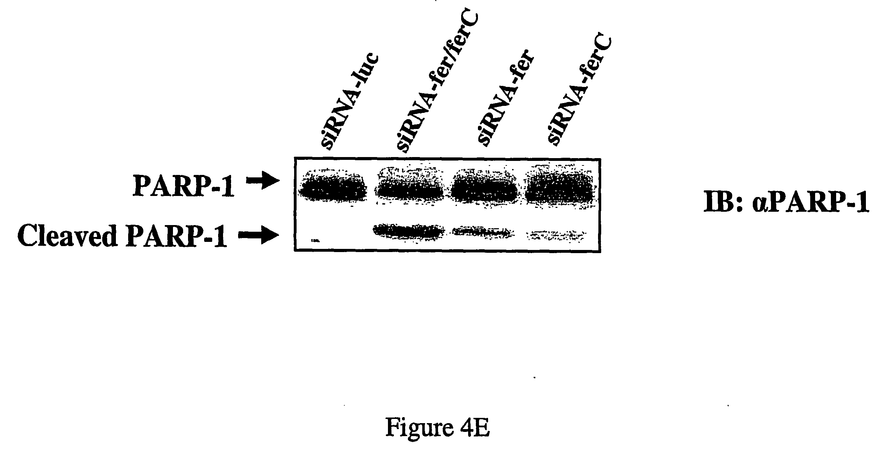

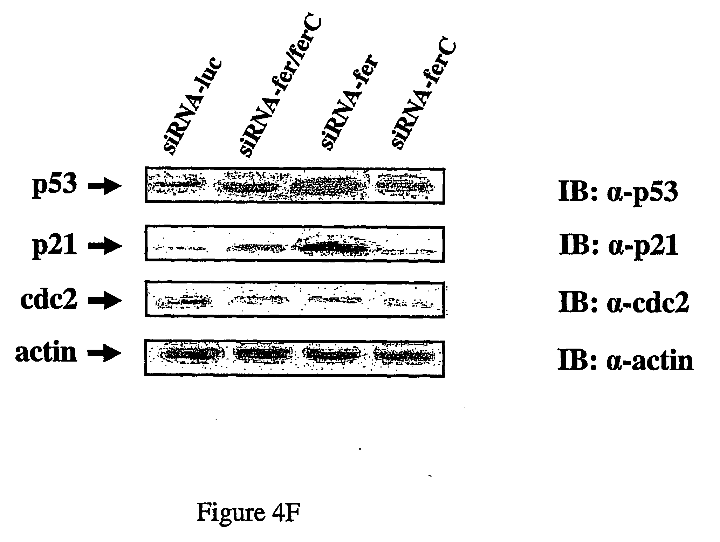

**Fig. 4** **A** shows the distribution of HCT116, and **Fig. 4** **B** shows the distribution of RKO cells, in the various cell-cycle fractions, upon treatment with siRNAs directed toward the fer and ferC RNAs; **Fig. 4** **C** shows incorporation of BrdU in CC cells following down-regulation of Fer or FerC; **Fig. 4** **D** shows staining of HCT116 Cells (2×10⁵) with Annexin V-FITC and propidium iodide after transfection with the siRNAs;
**Fig. 4** **E** shows lysates of HCT116 cells transfected with different siRNAs; and **Fig. 4** **F** shows resolution of whole cell protein lysates from Fer and FerC depleted HCT116 cells, which were resolved by SDS-PAGE and reacted with anti-p53, anti- p21, anti-cdc2 anti-actin antibodies, in a western blot analysis.

### EXPERIMENTAL RESULTS

Seven human CC cell-lines derived from various tumor stages, and from normal, human colonic epithelial cells (CCD33) were obtained from the ATCC and grown according to the ATCC instructions. Whole cell lysates were prepared from the cells and resolved by 10% SDS-PAGE (30 µg protein from each sample) and were then reacted with specific, anti-SH2-Fer antibodies (Priel-Halachmi et al., 2000) using western blot analysis.

Fig. 1 shows the expression profile of Fer in CC cells. In Fig. 1A, shows the western blot analysis of the normal human colon epithelial cells-CCD33 (lane 1), and from the seven human CC-lines (lanes 2-8), as indicated in Fig. 1A. In six of the CC cell-lines analyzed, an additional protein of about 47kDa molecular weight reacted with the specific, anti-SH2-Fer antibodies. The 47kDa was not detected in either the HT29 adenocarcinoma cells, or in the CCD33 normal colon epithelial cells. This 47kDa protein was also absent from normal human and mouse fibroblasts (data not shown).

To gain an indication whether the 47kDa protein represents a truncated variant of Fer, lysates from the CC cells were reacted with anti-Fer antibodies directed toward either the N-terminal tail or the C-terminal end of the protein. In Fig. 1B, lysates from HT29 and from HCT116 cells were reacted with antibodies directed toward the unique N-terminal tail of Fer (1-189 aa) (left panel), or antibodies directed toward the C-terminal end of Fer (right panel). α-actin served as protein quantity control (anti-actin, Sigma). While anti-C terminus antibodies reacted with the 47kDa protein, the anti-N terminal antibodies reacted only with the 94kDa full length Fer. These results suggested the accumulation in CC cells of a truncated variant of Fer, which lacks the N-terminal tail of the somatic kinase. This Fer variant is referred to herein as "*FerC*" (Fer colorectal cancer).

To examine whether the presence of FerC in CC cell-lines reflects the accumulation of this protein in a tumor specific manner, whole cell lysates from normal colon, primary CC tumors and from lung, liver and ovarian metastases of CC tumors were resolved by SDS-PAGE and were then reacted with anti-SH2-Fer antibodies. Lysates from primary colonic tissues or tumors were purchased from Origene Inc. (origene.com-HCRT1). Fig. 1C shows detection in lysates with anti-SH2-Fer antibodies of: HCT116 whole cell lysates (lane 1); normal human colon tissue (lanes 2,3); stage II primary colorectal adenocarcinoma (lanes 4,5); CC metastases to the liver (lane 6); CC metastases to the lung (lane 7); CC metastases to the ovary (lane 8); stage I adenocarcinoma (lane 9). The anti-SH2-Fer antibodies detected the p94Fer in all seven CC cell-lines as well as in the normal CCD33 cells. FerC was not detected in normal colon tissues nor in a stage I colon adenocarcinoma lysate. However, FerC was detected in primary stage II CC tumors. Furthermore, FerC was also present in CC metastases to the liver, lungs and ovaries.

In order to characterize and identify the Fer-related 47kD protein, RNAs containing Fer-related sequences were cloned from HCT116 and HT29 cells. RACE was performed by using a 5'/3' RACE kit (2nd generation, Roche) following the manufacturer's instructions. The specific primers used were:
Sp1: 5'-TCCCTTGCCCAGTAATTCTCCCAATATGAC-3' (SEQ. ID NO. 1)
Sp2: 5'-AACCCAGTGCCCTCGAATCGATAC-3' (SEQ. ID NO. 2)
Sp3: 5'-GGACATATTCACCAGGTTTCCCATGACTCTC-3' (SEQ. ID NO. 3).

The RACE product was analyzed on a 1% ethidium bromide stained agarose gel, and was cloned into the pGEM-T easy vector system (Promega). Tthe nucleotide sequence of the ferC cDNA is shown in SEQ ID NO: 11, and the longest open reading frame (ORF) translation product is shown in SEQ ID NO: 12. The unique N-terminal sequence is shown in SEQ ID NO: 13. The longest ORF of the ferC cDNA from HCT116 cells was found to encode a truncated Fer protein. FerC is 453 aa in length and has a calculated MW of 51kDa, though it migrates in PAGE as a 47kDa protein. FerC contains intact SH2 and kinase domains of Fer, which are linked to a unique 43 amino acid long N-terminal tail.

Fig. 2A shows schematically the structure of the FerC **25** protein in comparison to p94 Fer **26**. The unique N-terminal tail **27**, the SH2 domain **28** and the kinase domain (KD) **29** are depicted. Exons of the fer locus encoding the Fer and FerC proteins are indicated by the cubes **30**, which are numbered according to the exon number, and correspond to the encoded domains of FerC. Alignment of the ferC cDNA sequence with the human fer locus indicated that the unique N-terminal tail of FerC is encoded by intron 10 of the fer gene. The first nucleotide of the translation initiation codon of the ferC mRNA is transcribed from nucleotide 26,813 in intron **32** of the human fer locus. This intron is comprised of 48,281 nucleotides [ensemble human gene view]. The remainder of the FerC protein is encoded by exons 11-20 of the fer gene.

To confirm the link between the cloned ferC RNA and the 47kDa FerC protein, a semi-quantitative RT-PCR analysis was performed on RNA prepared from HCT116 and from HT29 cells. This was carried out using a 3' primer which is common to fer and ferC, and a unique primer corresponding to the 5' end of the ferC cDNA. RNA was extracted from HT29 and from HCT116 cells using TRI Reagent (Mol. Res. Center, Aurora, OH) following the manufacturer's instructions. 1µg total RNA was reverse-transcribed using the SuperScript first-strand synthesis system for RT-PCR (Invitrogen).

For semi-quantitative RT-PCR analysis, the PCR product was amplified using 35 cycles with the following primers derived from the human ferC cDNA:
5'-CCACATCAGAAGTCCACAGAGATCAGGAAAG-3' (SEQ. ID NO. 4)
5'-GCCCGCGAATTCACACTCAAAAGAGAACTAC-3' (SEQ. ID NO. 5)

The following gapdh RNA served as an internal control:
5'-AAGGTCATCCCTGAGCTGAACG-3' (SEQ. ID NO. 6)
5'-CAAAGGTGGAGGAGTGGGTGTC-3' (SEQ. ID NO. 7).

PCR products were separated on a 1% agarose gel and stained with ethidium bromide.

Fig. 2B shows, consistent with the accumulation of FerC in HCT116 cells, the existence of an RT-PCR product of 1420 bp and demonstrates the preferential accumulation of the ferC mRNA in HCT116 cells.

To gain insight into the function of the Fer proteins in CC cells, the Fer and FerC were simultaneously or individually knocked-down in the colon carcinoma cell-lines HCT116 and RKO, which express both the intact Fer and the truncated FerC.

Fer or FerC was selectively knocked down in HCT116 and RKO CC cells using 50nM specific and selective siRNAs directed toward the unique 5' sequences of the fer (siRNA-fer:5'-ACGUAUCCAAGUCUUGGCUACUUAU-3', SEQ. ID NO. 8) or ferC (siRNA-ferc:5'-CAGCUCUGAGCCUUCCACAUCAGAA-3', SEQ. ID NO. 9). A fer-specific siRNA directed towards a common sequence present in the fer and ferC RNAs (siRNA-fer/ferC: 5'-GCCCUAAGUUCAGUGAACUUCAGAA-3', SEQ. ID NO. 10) was used for simultaneous knockdown of Fer and FerC. A sequence targeting luciferase (siRNA-luc) was used as a non-relevant siRNA negative control (Dharmacon). 2×10⁵ cells were transfected with siRNAs using the Lipofectamine 2000 reagent, according to the manufacturer's instructions (Invitrogen). As shown in Figs. 3A and 3B, lanes 3 and 4, the specific siRNAs directed toward the unique 5' sequences of the fer and ferC mRNAs selectively knocked-down Fer or FerC, respectively.

Cell viability in HCT116 cell cultures following depletion of one or both of the Fer and FerC proteins was studied. HCT116 Cells were seeded in six well plates (2×10⁵ cells per well). 48h after transfection with the different siRNAs, cell viability was evaluated by using the XTT cell viability kit (Biol. Ind., Israel) following the manufacturer's instructions. The absorbance at 450 nm was measured using a microplate reader (Spectra Fluor Plus- Tecan Inc.). Statistical analysis was performed using the paired and unpaired Student's *t*-test, with a P value ≤0.05 being considered significant. The results are shown in Fig. 3C as mean ± standard error (SE) of the mean for 8 samples. Simultaneous knockdown of Fer and FerC in HCT116 cells decreased by two fold the percentage of viable cells in the treated cultures. Similar results were obtained upon knockdown of the Fer proteins in RKO cells (data not shown).

To determine whether Fer and FerC support the proliferation or survival of CC cells, cultures treated with siRNAs directed toward the fer and ferC mRNAs were subjected to flow-cytometric analysis. HCT116 cells were transfected with siRNAs for 48h and the percentage of cells incorporating BrdU was determined. Cell cycle analysis and BrdU incorporation assays were performed as described in Pasder et al., 2006.

The distribution of HCT116 and RKO cells in the various cell-cycle fractions, upon treatment with siRNAs directed toward the fer and ferC RNAs is shown in Figs. 4A and 4B. Changes in the sub-G0/G1 fraction are shown, as well. Selective knockdown of Fer or FerC decreased the percentage of cells residing in the S phase and increased the fraction of G2/M cells. This effect was more profound in Fer-depleted cells and was not enhanced upon the simultaneous knockdown of the two proteins. Notably, although the percentage of S phase cells was significantly decreased upon the knockdown of Fer or FerC, there was no significant increase seen in the G0/G1 fraction of the treated cells. This most probably is a result of the significant increase in the sub-G1 fraction of cells depleted of Fer or FerC, and could reflect a transition of G2/M arrested cells to apoptotic death rather than to the G0/G1 phase (Gascoigne and Taylor, 2008). Furthermore, the significant increase of the sub-G1 fraction upon the combined depletion of Fer and FerC could explain the lack of additive effects on the cell-cycle profile by the simultaneous knockdown of the two Fer proteins.

To substantiate the attenuation of the G1-S transition as a result of the knockdown of Fer or FerC, the percentage of BrdU incorporating cells was determined following Fer or FerC depletion. The results, shown in Fig. 4C, demonstrated a significant reduction in the level of BrdU incorporation upon the knockdown of Fer or FerC. Thus, the two Fer variants sustain the progression of both the Gl-S and G2-M transition points in CC cells.

As noted above, knockdown of Fer or FerC not only interfered with cell-cycle progression in HCT116 and RKO cells, but also induced apoptotic cell death. This effect was manifested by increased accumulation of the sub-G1 population in the treated cells, an effect that was enhanced upon the simultaneous knockdown of Fer and FerC (Figs. 4A and 4B). The profound induction of apoptotic death upon the simultaneous knock-down of Fer and FerC in CC cells was further demonstrated by staining the treated cells with Annexin V, which stains membranes of apoptotic cells (van Engeland et al., 1996) and by the identification of cleaved Poly(ADP-ribose) polymerase-1 (PARP-1) (Kaufmann et al., 1993) in the Fer and FerC depleted cells. For Annexin V staining, HCT116 Cells were plated in six well plates (2x105 cells per well), and 48h after transfection with siRNA were stained with Annexin V-FITC and propidium iodide using the MEBCYTO-apoptosis kit (MBL) following the manufacturer's instructions. The total cellular DNA content and bound Annexin V-FITC were determined using a Becton Dickinson flow cytometer (FACS Calibur), and all data were analyzed using the Cell Quest Pro software. The fractions of cells stained with both Annexin and propidium iodide (PI) is shown in the left histogram of Fig. 4D, and the fractions of cells stained with Annexin but not with PI is shown in the right histogram. Values represent means ±SE of Annexin-V staining in four independent experiments.

For the identification of cleaved PARP-1, lysates were resolved by SDS-PAGE and were then reacted with anti-PARP-1 antibody (Santa-Cruz) in a western-blot analysis. The results are shown in Fig. 4E, which represents one out of three independent experiments which gave similar results. Migration distances of the intact and cleaved PARP-1 are shown.

To unveil whether the apoptotic death invoked in the Fer and FerC depleted cells is linked to the induction of the transcription factor p53, the level of p53 was compared in the various treated cells. Fer and FerC were separately or simultaneously knocked-down in HCT116 cells with the different siRNAs. Lysates were prepared and resolved in SDS-PAGE and reacted with anti-p53, anti- p21, anti-cdc2 anti-actin antibodies (Santa-Cruz), in a western blot analysis. As shown in Fig. 4F, knock-down of Fer significantly up-regulated the level of p53 and concomitantly also the level of its down-stream effector p21, which acts as a negative regulator of proliferation and apoptosis in HCT116 cells (Polyak et al., 1996). In parallel, the level of another target of p53, the G2/M transition regulator cdc2, was decreased. However, simultaneous depletion of Fer and FerC interfered with the accumulation of p53 and its effects on the down-stream targets p21 and cdc2, thus allowing a shift from cell-cycle arrest to an increase in apoptotic death. Fig 4E represents one out of three independent experiments which gave similar results.

## Claims

1. A nucleotide sequence comprising a sequence that is antisense to at least a portion of the nucleotide sequence SEQ ID NO: 13 and capable of specifically binding to SEQ will NO. 13; **characterized in that** the sequence is SEQ ID NO: 9.

2. A pharmaceutical composition comprising a nucleotide sequence according to Claim 1, and a physiologically acceptable carrier.

3. The pharmaceutical composition according to Claim 2 further comprising a nucleotide sequence comprising a sequence that is antisense to at least a portion of the *fer* gene.

4. The pharmaceutical composition according to Claim 3 wherein the sequence that is antisense to at least a portion of *the fer* gene is SEQ ID NO 8.

5. The pharmaceutical composition according to any one of Claims 2 to 4 for use in the treatment of cancer.

6. The pharmaceutical composition according to any one of Claims 2 to 4 for use in the treatment of colorectal cancer.

7. The pharmaceutical composition according to any one of Claims 2 to 4 for use in the treatment of liver cancer.

8. An *in vitro* method for identifying a cancerous state in a cell comprising detecting FerC in the cell.

9. The method according to Claim 8 wherein the cancer is colorectal cancer.

10. The method according to Claim 8 or 9 comprising:
(a) extracting proteins from the cell;
(b) detecting Fer94 in the protein extract using a first antibody reactive with Fer94 and FerC;
(c) detecting Fer94 in the protein extract using a second antibody reactive with Fer94 and not reactive with FerC;
the presence of a protein in the extract reactive to the first antibody and not reactive to the second antibody being indicative of the presence of FerC in the cell.

11. The method according to Claim 10 wherein the second antibody binds to an epitope located at the N-terminus of Fer94.

12. A kit for identifying a cancerous state in a cell comprising:
(a) a first antibody reactive with Fer94 and FerC; and
(b) a second antibody reactive with Fer94 and not reactive with FerC.

## Patentansprüche

1. Nukleotidsequenz, die eine Sequenz umfasst, die zu mindestens einem Teil der Nukleotidsequenz SEQ.-ID-Nr. 13 antisense (gegensinnig) ist und spezifisch zur SEQ.-ID-Nr. 13 binden kann; **dadurch gekennzeichnet, dass** die Sequenz SEQ.-ID-Nr. 9 ist.

2. Pharmazeutische Zusammensetzung, die eine Nukleotidsequenz nach Anspruch 1 und einen physiologisch verträglichen Träger umfasst.

3. Pharmazeutische Zusammensetzung nach Anspruch 2, die weiter eine Nukleotidsequenz umfasst, die eine Sequenz umfasst, die zu mindestens einem Teil des fer-Gens antisense ist.

4. Pharmazeutische Zusammensetzung nach Anspruch 3, worin die Sequenz, die zu mindestens einem Teil des fer-Gens antisense ist, SEQ.-ID-Nr. 8 ist.

5. Pharmazeutische Zusammensetzung nach einem der Ansprüche 2 bis 4 zur Verwendung bei der Behandlung von Krebs.

6. Pharmazeutische Zusammensetzung nach einem der Ansprüche 2 bis 4 zur Verwendung bei der Behandlung von Kolorektalkrebs.

7. Pharmazeutische Zusammensetzung nach einem der Ansprüche 2 bis 4 zur Verwendung bei der Behandlung von Leberkrebs.

8. In-vitro-Verfahren zur Identifizierung eines kanzerösen Zustands in einer Zelle, das den Nachweis von FerC in der Zelle umfasst.

9. Verfahren nach Anspruch 8, worin der Krebs Kolorektalkrebs ist.

10. Verfahren nach Anspruch 8 oder 9, das Folgendes umfasst:
(a) Extrahieren von Proteinen aus der Zelle;
(b) Nachweisen von Fer94 in dem Proteinextrakt unter Verwendung eines ersten Antikörpers, der mit Fer94 und FerC reaktiv ist;
(c) Nachweisen von Fer94 in dem Proteinextrakt unter Verwendung eines zweiten Antikörpers, der mit Fer94 reaktiv ist und mit FerC nicht reaktiv ist;
wobei die Gegenwart eines Protein in dem Extrakt, das mit dem ersten Antikörper reaktiv ist und mit dem zweiten Antikörper nicht reaktiv ist, die Gegenwart von FerC in der Zelle anzeigt.

11. Verfahren nach Anspruch 10, worin der zweite Antikörper zu einem Epitop bindet, das am N-Terminus von Fer94 lokalisiert ist.

12. Kit zur Identifizierung eines kanzerösen Zustands in einer Zelle, der Folgendes umfasst:
(a) einen ersten Antikörper, der mit Fer94 und FerC reaktiv ist; und
(b) einen zweiten Antikörper, der mit Fer94 reaktiv ist und mit FerC nicht reaktiv ist.

## Revendications

1. Séquence nucléotidique comprenant une séquence qui est antisens par rapport à au moins une portion de la séquence nucléotidique SEQ ID n°13 et capable de se fixer spécifiquement à SEQ ID n°13, **caractérisée en ce que** la séquence est SEQ ID n°9.

2. Composition pharmaceutique comprenant une séquence nucléotidique selon la revendication 1, et un véhicule physiologiquement acceptable.

3. Composition pharmaceutique selon la revendication 2, comprenant en outre une séquence nucléotidique comprenant une séquence qui est antisens par rapport à au moins une portion du gène *fer.*

4. Composition pharmaceutique selon la revendication 3, dans laquelle la séquence qui est antisens par rapport à au moins une portion du gène *fer* est SEQ ID n°8.

5. Composition pharmaceutique selon l'une quelconque des revendications 2 à 4, pour une utilisation dans le traitement du cancer.

6. Composition pharmaceutique selon l'une quelconque des revendications 2 à 4, pour une utilisation dans le traitement du cancer colorectal.

7. Composition pharmaceutique selon l'une quelconque des revendications 2 à 4, pour une utilisation dans le traitement du cancer du foie.

8. Méthode *in vitro* pour l'identification d'un état cancéreux dans une cellule, comprenant la détection de FerC dans la cellule.

9. Méthode selon la revendication 8, dans laquelle le cancer est le cancer colorectal.

10. Méthode selon la revendication 8 ou 9, comprenant :
(a) l'extraction de protéines à partir de la cellule ;
(b) la détection de Fer94 dans l'extrait de protéines à l'aide d'un premier anticorps réactif avec Fer94 et FerC ;
(c) la détection de Fer94 dans l'extrait de protéines à l'aide d'un deuxième anticorps réactif avec Fer94 et non réactif avec FerC ;
la présence d'une protéine dans l'extrait réactive avec le premier anticorps et non réactive avec le deuxième anticorps constituant une indication de la présence de FerC dans la cellule.

11. Méthode selon la revendication 10, dans laquelle le deuxième anticorps se fixe à un épitope situé à l'extrémité N-terminale de Fer94.

12. Kit d'identification d'un état cancéreux dans une cellule, comprenant :
(a) un premier anticorps réactif avec Fer94 et FerC ; et
(b) un deuxième anticorps réactif avec Fer94 et non réactif avec FerC.


### SEQUENCE LISTING

<110> UNIFER LTD
   URIFER LTD
<120> NOVEL FER -LIKE PROTEIN, PHARMACEUTICAL COMPOSITIONS CONTAINING IT, AND METHOD FOR ITS PREPARATION AND USE
<130> 1893544
<160> 14
<170> PatentIn version 3.1
<210> 1
   <211> 30
   <212> DNA
   <213> Homo sapiens
<400> 1
   tcccttgccc agtaattctc ccaatatgac 30
<210> 2
   <211> 24
   <212> DNA
   <213> Homo sapiens
<400> 2
   aacccagtgc cctcgaatcg atac 24
<210> 3
   <211> 31
   <212> DNA
   <213> Homo sapiens
<400> 3
   ggacatattc accaggtttc ccatgactct c 31
<210> 4
   <211> 31
   <212> DNA
   <213> Homo sapiens
<400> 4
   ccacatcaga agtccacaga gatcaggaaa g 31
<210> 5
   <211> 31
   <212> DNA
   <213> Homo sapiens
<400> 5
   gcccgcgaat tcacactcaa aagagaacta c 31
<210> 6
   <211> 22
   <212> DNA
   <213> Homo sapiens
<400> 6
   aaggtcatcc ctgagctgaa cg 22
<210> 7
   <211> 22
   <212> DNA
   <213> Homo sapiens
<400> 7
   caaaggtgga ggagtgggtg tc 22
<210> 8
   <211> 25
   <212> RNA
   <213> Homo sapiens
<400> 8
   acguauccaa gucuuggcua cuuau 25
<210> 9
   <211> 25
   <212> RNA
   <213> Homo sapiens
<400> 9
   cagcucugag ccuuccacau cagaa 25
<210> 10
   <211> 25
   <212> RNA
   <213> Homo sapiens
<400> 10
   gcccuaaguu cagugaacuu cagaa 25
<210> 11
   <211> 1420
   <212> DNA
   <213> Homo sapiens
<400> 11
   ttggtaaatt gacttccttg ggcccactgt cactatctct tagctaaatt ttgagatgga     60
   gcagaaaatg aaatgtcctc attgtaagga tcagctggag tctggtttcg gctcccaatc    120
   ctgtaaaaca tgtgctttaa tgttcagctc tgagccttcc acatcagaag tccacagaga    180
   tcaggaaaga aaggagaggc tatccaaatt tgaatctatt cgtcattcaa ttgctggaat    240
   aattaggtct ccaaaatctg cactgggctc ttcagcactt tctgatatga tctccatcag    300
   tgagaagcct ttggcagaac aggactggta ccatggtgca attcccagaa tagaagctca    360
   agaactgtta aaaaaacaag gagacttttt ggtgcgagag agtcatggga aacctggtga    420
   atatgtcctt tctgtatatt ctgatggaca gaggagacat tttatcatac aatatgttga    480
   taacatgtat cgattcgagg gcactgggtt ttcaaacatt cctcaactta tagatcatca    540
   ctatacaaca aaacaggtca tcactaagaa atcaggtgta gttctgctga atcctattcc    600
   taaggacaag aaatggattc tcagtcatga agatgtcata ttgggagaat tactgggcaa    660
   gggaaatttt ggtgaagtat ataagggcac attaaaggat aaaacttctg ttgctgttaa    720
   aacatgtaaa gaagatcttc ctcaggaatt gaaaataaaa tttttacaag aagccaaaat    780
   tctcaagcaa tatgatcatc ccaatattgt caaacttata ggagtttgca cacaaagaca    840
   gcctgtctac atcattatgg aactggtttc aggaggtgat ttcctcacct ttctgagaag    900
   gaagaaggat gaactaaaac tcaaacagtt agtgaaattt tcattagacg ctgctgctgg    960
   tatgttgtat ctcgagagta aaaactgtat acacagggac cttgctgcaa gaaactgcct   1020
   ggtaggtgaa aataatgttc tgaaaatcag tgactttgga atgtctcgtc aagaggatgg   1080
   tggagtgtat tcatcttctg gcttaaagca gattcccatt aaatggacag caccggaagc   1140
   tcttaattat gggagataca gttcagagag tgacgtgtgg agctttggca tccttctctg   1200
   ggagaccttc agcttagggg tttgtccgta ccctggaatg acaaatcagc aagcaagaga   1260
   gcaagtagaa agaggatacc ggatgtcagc tccccagcac tgtccagagg atatttccaa   1320
   aatcatgatg aagtgttggg attataaacc tgaaaatcgc cctaagttca gtgaacttca   1380
   gaaagagctc actatcatca agagaaaact cacatagtga                         1420
<210> 12
   <211> 453
   <212> PRT
   <213> Homo sapiens
<400> 12
<210> 13
   <211> 129
   <212> DNA
   <213> Homo sapiens
<400> 13
   atggagcaga aaatgaaatg tcctcattgt aaggatcagc tggagtctgg tttcggctcc     60
   caatcctgta aaacatgtgc tttaatgttc agctctgagc cttccacatc agaagtccac    120
   agagatcag                                                            129
<210> 14
   <211> 43
   <212> PRT
   <213> Homo sapiens
<400> 14
   Met Glu Gln Lys Met Lys Cys Pro His Cys Lys Asp Gln Leu Glu Ser
   1               5                   10                  15
   Gly Phe Gly Ser Gln Ser Cys Lys Thr Cys Ala Leu Met Phe Ser Ser
               20                  25                  30
   Glu Pro Ser Thr Ser Glu Val His Arg Asp Gln
           35                  40